# EUROPEAN PATENT APPLICATION

(11) **EP 1 563 806 A1**
(43) Date of publication of application: **17.08.2005**
(21) Application number: 05250802.5
(22) Date of filing: 11.02.2005
(51) Int. Cl.: A61F 2/06

(54) **Connector members for stents**

(30) Priority: 13.02.2004 US 779493
(71) Applicant: Nitinol Development Corporation, Fremont, California 94539 (US)
(72) Inventor: Bonsignore, Craig, San Jose, California 95112 (US); Duerig, Thomas W., Fremont California 94539 (US); Carlson, John, Morrow Ohio 45152 (US)
(74) Representative: Fisher, Adrian John

(57) **Abstract**

Accordingly, it is a object of the invention to create a stent which comprises structurally strong radial rings which are connected by structurally weak connectors. These connectors then separate within the body so that they are able to cause the stent to be emplaced exclusively at selected points within the lumen with a clear separation made between each of these radial rings.

## Description

### BACKGROUND OF THE INVENTION

Historically, stents have been designed to remain contiguous within the body. However, there may be instances where it may be desirable to have a stent which is separable within the body. For instances, in vessels which may be subject to longitudinal elongation or excessive compression or bending, a frangible stent may prove useful for good vessel opposition. Or, at a bifurcation, it may be useful to insure that the expanded stent does not migrate into the lumen area. The cyclic strains which propagate though the structure of the stent can potentially cause greater damage to the stent. And may be avoided by having the stent become physically separable within the body.

Accordingly, it is an object of the invention to create a stent which comprises structurally strong radial rings which are connected by structurally weak connectors. These connectors then separate within the body so that they are able to cause the stent to be emplaced exclusively at selected points within the lumen with a clear separation made between each of these radial rings.

### BRIEF DESCRIPTION OF THE DRAWINGS

Figures 1A, 1B, and 1C are stents showing a frangible section contained in its connector members.
Figures 2A, 2B, and 2C are stents which contains polymeric bridges adjoining adjacent metallic rings within the stent.

### DETAILED DESCRIPTION OF THE DRAWINGS

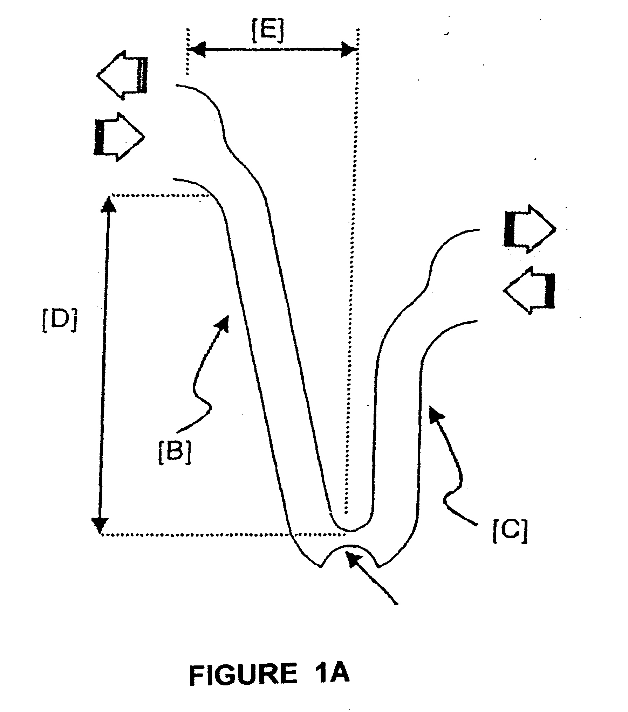

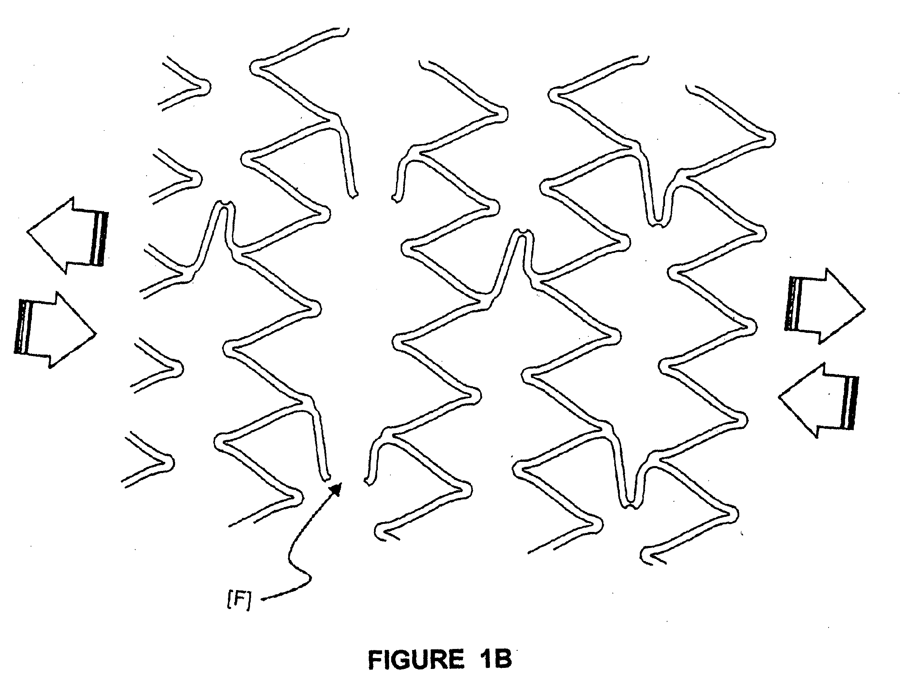

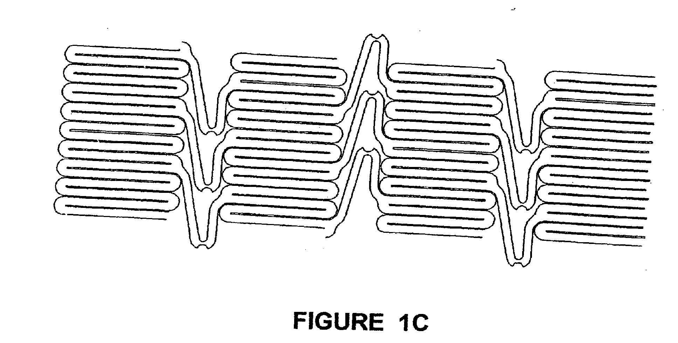

As seen in Figures 1A, 1B, and 1C there is described herein a stent 50 which comprises standard slotted radial rings 100. These rings may be of stainless steel or Nitinol, in a form much like the Palmaz™ or Palmaz-Schatz ™ stent made by Cordis Corporation or the Smart Stent™ also made by Cordis Corporation, Miami Lakes, Florida. These radial rings are intended to be of strong radial strength when emplaced within the body. They may be self-expanding or they may be expanded using a balloon catheter (not shown), so that their expansion is taken beyond the elastic limit of the material so that the stent rings take a permanent set within the body.

Importantly, the radial rings are connected by flexible connector 150 members spaced around the rings. As seen in the current figures, there are contained three connectors 150 per ring 120, however, it is well known to place multiple connector members and these connectors 150 may be placed as desired on the stent.

Importantly, about midway along a portion of the connector member 130 there is contained a position of weakness labeled as "A" on Figure 1A. When the stents are emplaced within the body, longitudinal motion of the lumen causes the stents 50 to expand and contract in the longitudinal direction, as seen by the arrows B drawn in Figure 1A and Figure 1B. This causes the notched strain limitor 160 to act as a focal point for the cyclic strain under the loading conditions when elements B and C are deflected in the direction of the arrows. Under these loading conditions, the structure is designed to experience a fatigue fracture in the notched area, A, rather than to communicate stresses or strains throughout the entire structure of stents 50, 50'. This can prevent potentially harmful cyclic strains from causing undesirable fatigue fractures in the radial support members.

It is noticed that it may be advantageous to maximize the length d and e of a connector 150 so as maximize the fulcrum applied at the section A. This will reduce the time in which it will take the connector member 150 to break apart so that the loads in which the stent is subjected to will be reduced.

During manufacture, the proposed stent of the current invention is made under typical conventional stent manufacturing methods. However, the notched design 130 may be laser cut or etched into the connector members 150 upon creation, so that during emplacement into the body the connector member is able to be broken as desired. Of course, the stent can be loaded with heparin or other drug coatings, as is now well appreciated in the art. The stent may be made from stainless steel or nitinol or any other biocompatible material.

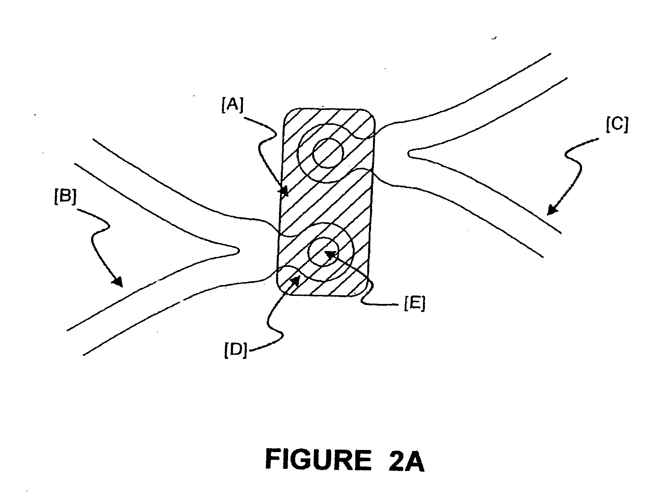

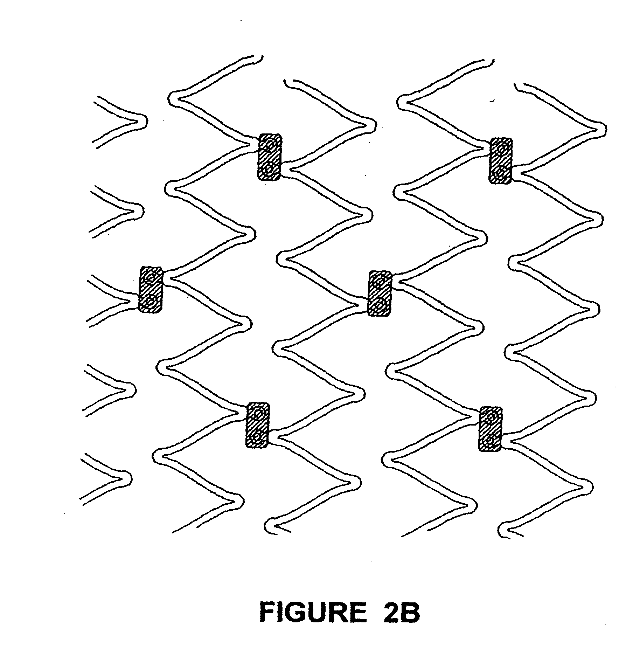

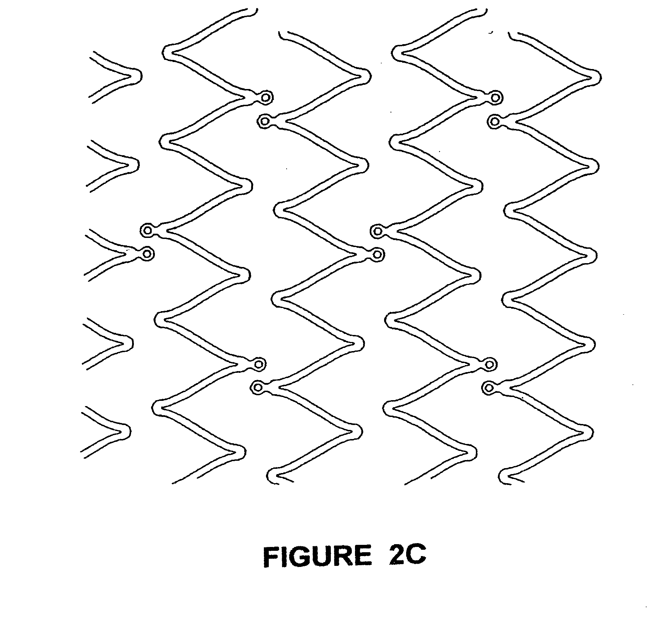

As seen in Figure 2A, 2B, and 2C there is contained an alternate embodiment of the current invention. Here, there are polymeric bridges 175 which are placed between the radial rings. The radial rings are quite similar to the radial rings of Figures 1A, 1B and 1C, except that there are contained protrusions F which protrude from either side of the radial rings 120 at a location where it may be desirable to connect one ring to the other ring. The polymeric bridge identified as 175, in Figure 2A, contains slots 180 in which the metallic tab G is emplaced. This tab G also contains a hole H which can be filled with polymer. In other words, during manufacture, the rings are first fashioned using standard cutting techniques, such as laser cutting or etching. The stent rings themselves are made of standard materials such as stainless steel, tantulum, titanium and nickel titanium alloys such as nitinol and the like. After their manufacture, the stent is placed so that the rings are juxtaposed one to the other as seen in Figure 2C. Thereafter, the polymeric bridges may be fused directly to the stents so that the polymer not only surrounds each of the tabs D, but fills the holes E upon manufacture. Thus, the polymer and the polymer that surrounds each of the tabs in multiple fashion so that the polymeric bridge remains integral prior to delivery into the body.

After delivery, the stents 50, 50' can be expanded using conventional expansion methods such as balloon catheters. Or, the stents may be a self-expanding. In either event, after the stents are expanded within the lumen, the polymeric bridges are subjected to standard corrosive forces located within the body. These corrosive forces cause the breakdown of the polymeric bridge after a certain period of time. This breakdown causes the rings to separate one from the other after a predetermined length of time. It is during this breakdown that the forces which may be caused by cyclic strains caused placed on the stent will become reduced as they only affect one particular ring in one particular location at a time.

Because the bridge acts as a flexible hinge, it also may improve deployment characteristics. This hinge may be somewhat more flexible during delivery than a standard connector member so that the stent may be able to obtain a position within a slightly more difficult lumens as compared to prior art stents. As constructed, the combined structure of the stent will act as a single stent during delivery and deployment. However, after the polymeric bridges are absorbed the metallic structures forming the rings become completely unconnected and independent of one another. This may be advantageous in vessels which may be subject to longitudinal elongation compressing or bending, as explained above.

Furthermore, when combined with polymer drug eluting technology, the polymeric bridge may actually provide an additional drug delivery reservoir for the stent. In fact, it may be possible to have a bolus of drug contained within the polymeric at tab E and thereafter delivered in one large dosage upon secretion of the polymeric material into the body.

Naturally, the stent of the present invention should only be understood in context of the attached claims and their equivalents which are appended as follows.

## Claims

1. A stent comprising:
a plurality of circumferential rings, said rings connected by connector members, and
the connector members designed to be frangible.

2. A stent comprising:
a plurality of circumferential rings, said rings connected by connector members, and
the connector members being flexible members containing an area of weakness.

3. A stent comprising:
a plurality of circumferential rings, said rings connected by connector members, and
the connector members being absorbable.

4. A stent comprising:
a plurality of circumferential rings, said rings connected by connector members, and
the connector members being attached to each ring only at selected points on the ring, and
the connector members being frangible.

5. The connector members being attached to each ring only at selected points on the ring, and
the connector members having flexible members containing an area of weakness.

6. The stent of claims 1 to 5 when the connector member is attached to the ring member at a selected portion on a ring member.

7. The stent of claim 1-6 where there is contained a weakened point in the connector member, said weakened point placed about midway between ring members.

8. The stent of claim 1-6 where the stent rings are frangible from one another at said connector member upon the application of a predetermined strain on the lumen of a vessel.
